# EUROPÄISCHE PATENTSCHRIFT

(11) **EP 0 923 534 B1**
(45) Veröffentlichungstag und Bekanntmachung des Hinweises auf die Patenterteilung: **04.10.2000**
(21) Anmeldenummer: 97932809.3
(22) Anmeldetag: 11.07.1997
(51) Int. Cl.: C07C 209/68, C07B 55/00

(54) **VERFAHREN ZUR HERSTELLUNG VON RACEMISCHEN PHENETHYLAMINEN**
PROCESS FOR PREPARING RACEMIC PHENETHYLAMINES
PROCEDE DE PREPARATION D'AMINES PHENETHYLIQUES RACEMIQUES

(30) Priorität: 23.07.1996 DE 19629692
(43) Veröffentlichungstag der Anmeldung: 23.06.1999
(73) Patentinhaber: BAYER AG, 51368 Leverkusen (DE)
(72) Erfinder: STELZER, Uwe, D-51399 Burscheid (DE)
(86) Internationale Anmeldenummer: EP9703691
(87) Internationale Veröffentlichungsnummer: WO9803465

(56) Entgegenhaltungen:
- EP-A- 0 489 682
- PATENT ABSTRACTS OF JAPAN vol. 095, no. 010, 30.November 1995 & JP 07 188120 A (TORAY IND INC), 25.Juli 1995, in der Anmeldung erwähnt
- PATENT ABSTRACTS OF JAPAN vol. 096, no. 005, 31.Mai 1996 & JP 08 027073 A (AJINOMOTO CO INC), 30.Januar 1996,

## Beschreibung

Die vorliegende Erfindung betrifft ein neues Verfahren zur Herstellung von bekannten Phenethylaminen durch Racemisierung von optisch aktiven Phenethylaminen.

Es sind bereits mehrere Verfahren zur Herstellung optisch aktiver Phenethylamine bekannt (vgl. EP-A 0 341 475). Dabei werden die racemischen Ausgangsprodukte durch Zugabe von Hilfsreagenzien in die einzelnen Enantiomeren zerlegt. Um diese bekannten Verfahren zur Racematspaltung wirtschaftlich zu gestalten, muß das jeweils unerwünschte Enantiomere wieder racemisiert und in den Kreislauf zurückgeführt werden.

In diesem Zusammenhang wurden schon mehrere Methoden beschrieben, die geeignet sind, um nicht benötigte Enantiomere von Phenethylaminen zu racemisieren. So lassen sich optisch aktive Phenethylamine durch Behandlung mit Alkanolaten in Gegenwart von Dimethylsulfoxid in die entsprechenden Racemate überführen (vgl. EP-A 0 489 682). Das Verfahren wird allerdings dadurch beeinträchtigt, daß die als Hilfsreagenzien dienenden Alkanolate relativ kostspielig sind.

Weiterhin ist bereits bekannt geworden, daß sich racemische Phenethylamine herstellen lassen, indem man optisch aktive Phenethylamine mit Acetophenon-Derivaten umsetzt, die dabei entstehenden optisch aktiven Schiffschen Basen dann mit Hilfe von Kalium-tert.-butanolat racemisiert und die dadurch entstehenden racemischen Schiffschen Basen mit Säuren spaltet (vgl. JP-A 07-188 120 und Derwent Abstract Nr. 95-290 356/38). Nachteilig an diesem Verfahren ist jedoch, daß die Ausbeuten an den gewünschten Racematen nicht immer befriedigend sind. Ungünstig ist außerdem, daß zur Racemisierung das teure Kalium-tert.-butanolat erforderlich ist.

Es wurde nun gefunden, daß man racemische Phenethylamine der Formel 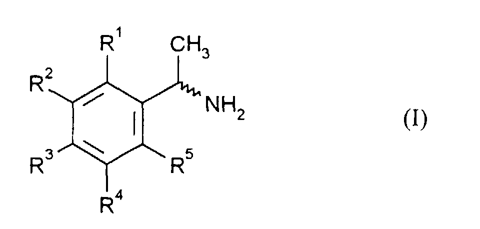 in welcher
- R¹, R², R³, R⁴ und R⁵: unabhängig voneinander für Wasserstoff, Halogen, Cyano, Nitro, Alkyl, Alkoxy, Alkylthio, Alkylsulfinyl, Alkylsulfonyl, Dialkylamino, Halogenalkyl, Halogenalkoxy, Halogenalkylthio, Halogenalkylsulfinyl oder Halogenalkylsulfonyl stehen,
erhält, wenn man
a) in einer ersten Stufe optisch aktive Phenethylamine der Formel 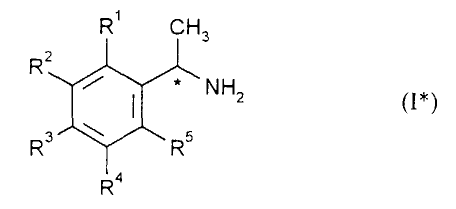 in welcher
   R¹, R², R³, R⁴ und R⁵ die oben angegebenen Bedeutungen haben,
   mit Acetophenon-Derivaten der Formel 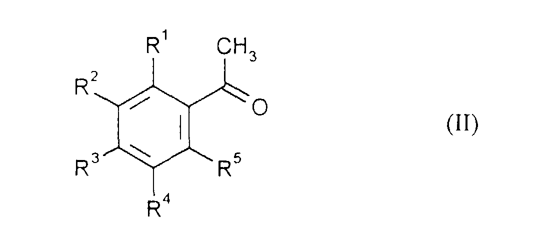 in welcher
   R¹, R², R³, R⁴ und R⁵ die oben angegebenen Bedeutungen haben,
   wobei das eingesetzte optisch aktive Phenethylamin und das eingesetzte Acetophenon-Derivat im Phenylteil jeweils identisch substituiert sind,
   gegebenenfalls in Gegenwart eines Verdünnungsmittels und gegebenenfalls in Gegenwart eines Katalysators umsetzt,
b) dann in einer zweiten Stufe die entstandenen optisch aktiven Schiffschen Basen der Formel 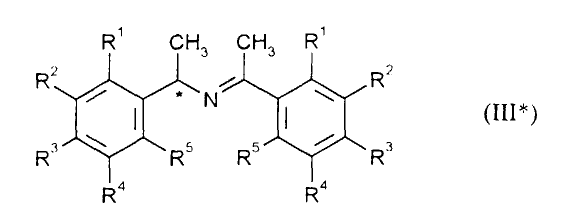 in welcher
   R¹, R², R³, R⁴ und R⁵ die oben angegebenen Bedeutungen haben,
   mit Metallhydroxiden, die einen Wassergehalt zwischen 0, 1 und 50 Gew.-% aufweisen, gegebenenfalls unter Schutzgasatmosphäre umsetzt und
c) in einer dritten Stufe die erhaltenen racemischen Schiffschen Basen der Formel 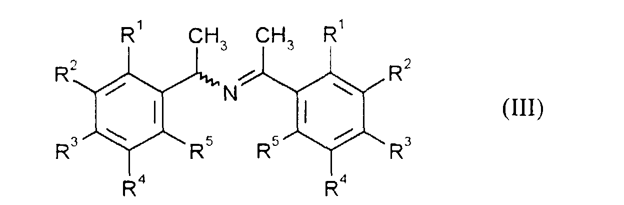 in welcher
   R¹, R², R³, R⁴ und R⁵ die oben angegebenen Bedeutungen haben,
   mit Säuren in Gegenwart von Wasser umsetzt.

Zur Kennzeichnung optisch aktiver Verbindungen ist das Chiralitätszentrum in den obigen Formeln und auch weiterhin jeweils durch (*) markiert.

Es ist als äußerst überraschend zu bezeichnen, daß sich racemische Phenethylamine der Formel (I) nach dem erfindungsgemäßen Verfahren in höherer Ausbeute herstellen lassen als nach dem ähnlichsten vorbeschriebenen Verfahren, bei dem optisch aktive Schiffsche Basen mit Hilfe von Kalium-tert.-butanolat racemisiert werden. Im übrigen mußte aufgrund des vorbekannten Standes der Technik angenommen werden, daß optisch aktive Phenethylamine der Formel (I*) bei der Behandlung mit wasserhaltigen Metallhydroxiden in erheblichem Maße zersetzt werden. Im Gegensatz zu den Erwartungen ist das aber nicht der Fall.

Das erfindungsgemäße Verfahren zeichnet sich durch eine Reihe von Vorteilen aus. So ermöglicht es die Herstellung von racemischen Phenethylaminen aus unerwünschten, bei Racematspaltungen zwangsläufig anfallenden Enantiomeren in sehr hoher Ausbeute. Günstig ist auch, daß sich sowohl (R)- als auch (S)-Enantiomere gleichermaßen gut racemisieren lassen und daß die Substituenten am Phenylring breit variierbar sind. Von Vorteil ist weiterhin, daß die Racemisierung mit Hilfe von preiswerten Metallhydroxiden gelingt. Schließlich bereitet auch die Durchführung der Umsetzungen und die Isolierung der gewünschten Substanzen keinerlei Schwierigkeiten.

Verwendet man (S)-1-(4-Chlor-phenyl)-ethylamin und 4-Chloracetophenon als Ausgangsstoffe, wäßriges Kaliumhydroxid als Racemisierungsreagenz und wäßrige Salzsäure zur Spaltung der racemischen Schiffschen Base, so kann der Verlauf des erfindungsgemäßen Verfahrens durch das folgende Formelschema veranschaulicht werden.

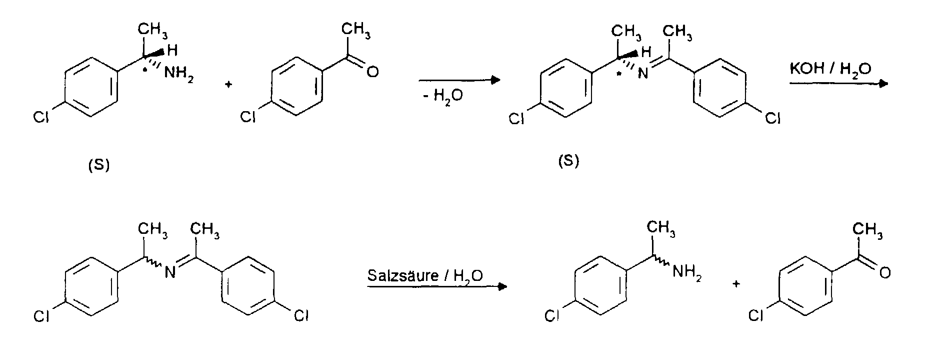

Die zur Durchführung des erfindungsgemäßen Verfahrens als Ausgangsstoffe benötigten optisch aktiven Phenethylamine sind durch die Formel (I*) allgemein definiert. Das Chiralitätszentrum kann entweder die (R)- oder die (S)-Konfiguration aufweisen.

Vorzugsweise verwendbar sind Phenethylamine der Formel (I*), in denen
- R¹, R², R³, R⁴ und R⁵: unabhängig voneinander für Wasserstoff, Fluor, Chlor, Brom, Cyano, Nitro, Alkyl mit 1 bis 4 Kohlenstoffatomen, Alkoxy mit 1 oder 2 Kohlenstoffatomen, Alkylthio mit 1 oder 2 Kohlenstoffatomen, Alkylsulfinyl mit 1 oder 2 Kohlenstoffatomen, Alkylsulfonyl mit 1 oder 2 Kohlenstoffatomen, Dialkylamino mit jeweils 1 oder 2 Kohlenstoffatomen in den Alkylketten, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Halogenalkylsulfinyl mit 1 oder 2 Kohlenstoffatomen und 1 bis 5 Halogenatomen oder Halogenalkylsulfonyl mit 1 oder 2 Kohlenstoffatomen und 1 bis 5 Halogenatomen stehen.

Besonders bevorzugt verwendbar sind Phenethylamine der Forme (I*), in denen
- R¹, R², R³, R⁴ und R⁵: unabhängig voneinander für Wasserstoff, Fluor, Chlor, Cyano, Nitro, Methyl, Ethyl, n- oder i-Propyl, Methoxy, Methylthio, Methylsulfinyl, Methylsulfonyl, Dimethylamino, Trifluormethyl, Trifluormethoxy, Difluormethoxy, Trifluormethylthio, Difluormethylthio, Trifluormethylsulfinyl, Difluormethylsulfinyl, Trifluormethylsulfonyl oder Difluormethylsulfonyl stehen.

Eine ganz besonders bevorzugt verwendbare Gruppe von optisch aktiven Phenethylaminen sind Stoffe der Formel (I*), in denen
- R⁴ und R⁵: für Wasserstoff stehen und
- R¹, R² und R³: unabhängig voneinander für Fluor, Chlor, Nitro, Methyl, Methoxy, Methylthio, Methylsulfinyl oder Methylsulfonyl stehen.

Eine weitere ganz besonders bevorzugt verwendbare Gruppe von optisch aktiven Phenethylaminen sind Stoffe der Formel (I*), in denen
- R¹ und R⁵: für Wasserstoff stehen und
- R², R³ und R⁴: unabhängig voneinander für Fluor, Chlor, Nitro, Methyl, Methoxy, Methylthio, Methylsulfinyl oder Methylsulfonyl stehen.

Eine weitere ganz besonders bevorzugt verwendbare Gruppe von optisch aktiven Phenethylaminen sind Stoffe der Formel (I*), in denen
- R³, R⁴ und R⁵: für Wasserstoff stehen und
- R¹ und R²: unabhängig voneinander für Fluor, Chlor, Nitro, Methyl, Methoxy, Methylthio, Methylsulfinyl oder Methylsulfonyl stehen

Eine weitere ganz besonders bevorzugt verwendbare Gruppe von optisch aktiven Phenethylaminen sind Stoffe der Formel (I*), in denen
- R¹, R⁴ und R⁵: für Wasserstoff stehen und
- R² und R³: unabhängig voneinander für Fluor, Chlor, Nitro, Methyl, Methoxy, Methylthio, Methylsulfinyl oder Methylsulfonyl stehen.

Eine weitere ganz besonders bevorzugt verwendbare Gruppe von optisch aktiven Phenethylaminen sind Stoffe der Formel (I*), in denen
- R², R⁴ und R⁵: für Wasserstoff stehen und
- R¹ und R³: unabhängig voneinander für Fluor, Chlor, Nitro, Methyl, Methoxy, Methylthio, Methylsulfinyl oder Methylsulfonyl stehen.

Eine weitere ganz besonders bevorzugt verwendbare Gruppe von optisch aktiven Phenethylaminen sind Stoffe der Formel (I*), in denen
- R², R³ und R⁴: für Wasserstoff stehen und
- R¹ und R⁵: unabhängig voneinander für Fluor, Chlor, Nitro, Methyl, Methoxy, Methylthio, Methylsulfinyl oder Methylsulfonyl stehen.

Eine weitere ganz besonders bevorzugt verwendbare Gruppe von optisch aktiven Phenethylaminen sind Stoffe der Formel (I*), in denen
- R¹, R³ und R⁵: für Wasserstoff stehen und
- R² und R⁴: unabhängig voneinander für Fluor, Chlor, Nitro, Methyl, Methoxy, Methylthio, Methylsulfinyl oder Methylsulfonyl stehen.

Eine weitere ganz besonders bevorzugt verwendbare Gruppe von optisch aktiven Phenethylaminen sind Stoffe der Formel (I*), in denen
- R², R³, R⁴ und R⁵: für Wasserstoff stehen und
- R¹: für Fluor, Chlor, Nitro, Methyl, Methoxy, Methylthio, Methylsulfinyl oder Methylsulfonyl steht.

Eine weitere ganz besonders bevorzugt verwendbare Gruppe von optisch aktiven Phenethylaminen sind Stoffe der Formel (I*), in denen
- R¹, R³, R⁴ und R⁵: für Wasserstoff stehen und
- R²: für Fluor, Chlor, Nitro, Methyl, Methoxy, Methylthio, Methylsulfinyl oder Methylsulfonyl steht.

Eine weitere ganz besonders bevorzugt verwendbare Gruppe von optisch aktiven Phenethylaminen sind Stoffe der Formel (I*), in denen
- R¹, R², R⁴ und R⁵: für Wasserstoff stehen und
- R³: für Fluor, Chlor, Nitro, Methyl, Methoxy, Methylthio, Methylsulfinyl oder Methylsulfonyl steht.

Die optisch aktiven Phenethylamine der Formel (I*) sind bekannt oder lassen sich nach bekannten Methoden herstellen (vgl. DE-A 43 32 738).

Die bei der Durchführung des erfindungsgemäßen Verfahrens als Reaktionskomponenten benötigten Acetophenon-Derivate sind durch die Formel (II) allgemein definiert. Es werden jeweils solche Acetophenon-Derivate eingesetzt, in denen die Substituenten R¹, R², R³, R⁴ und R⁵ die gleiche Bedeutung haben wie die entsprechenden Substituenten in den optisch aktiven Phenethylaminen der Formel (I*).

Vorzugsweise verwendbar sind Acetophenon-Derivate der Formel (II), in denen
- R¹, R², R³, R⁴ und R⁵: unabhängig voneinander für Wasserstoff, Fluor, Chlor, Brom, Cyano, Nitro, Alkyl mit 1 bis 4 Kohlenstoffatomen, Alkoxy mit 1 oder 2 Kohlenstoffatomen, Alkylthio mit 1 oder 2 Kohlenstoffatomen, Alkylsulfinyl mit 1 oder 2 Kohlenstoffatomen, Alkylsulfonyl mit 1 oder 2 Kohlenstoffatomen, Dialkylamino mit jeweils 1 oder 2 Kohlenstoffatomen in den Alkylketten, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Halogenalkylsulfinyl mit 1 oder 2 Kohlenstoffatomen und 1 bis 5 Halogenatomen oder Halogenalkylsulfonyl mit 1 oder 2 Kohlenstoffatomen und 1 bis 5 Halogenatomen stehen.

Besonders bevorzugt verwendbar sind Acetophenon-Derivate der Forme (II), in denen
- R¹, R², R³, R⁴ und R⁵: unabhängig voneinander für Wasserstoff, Fluor, Chlor, Cyano, Nitro, Methyl, Ethyl, n- oder i-Propyl, Methoxy, Methylthio, Methylsulfinyl, Methylsulfonyl, Dimethylamino, Trifluormethyl, Trifluormethoxy, Difluormethoxy, Trifluormethylthio, Difluormethylthio, Trifluormethylsulfinyl, Difluormethylsulfinyl, Trifluormethylsulfonyl oder Difluormethyl sulfonyl stehen.

Eine ganz besonders bevorzugt verwendbare Gruppe von Acetophenon-Derivaten sind Stoffe der Formel (II), in denen
- R⁴ und R⁵: für Wasserstoff stehen und
- R¹, R² und R³: unabhängig voneinander für Fluor, Chlor, Nitro, Methyl, Methoxy, Methylthio, Methylsulfinyl oder Methylsulfonyl stehen.

Eine weitere ganz besonders bevorzugt verwendbare Gruppe von Acetophenon-Derivaten sind Stoffe der Formel (II), in denen
- R¹ und R⁵: für Wasserstoff stehen und
- R², R³ und R⁴: unabhängig voneinander für Fluor, Chlor, Nitro, Methyl, Methoxy, Methylthio, Methylsulfinyl oder Methylsulfonyl stehen.

Eine weitere ganz besonders bevorzugt verwendbare Gruppe von Acetophenon-Derivaten sind Stoffe der Formel (II), in denen
- R³, R⁴ und R⁵: für Wasserstoff stehen und
- R¹ und R²: unabhängig voneinander für Fluor, Chlor, Nitro, Methyl, Methoxy, Methylthio, Methylsulfinyl oder Methylsulfonyl stehen.

Eine weitere ganz besonders bevorzugt verwendbare Gruppe von Acetophenon-Derivaten sind Stoffe der Formel (II), in denen
- R¹, R⁴ und R⁵: für Wasserstoff stehen und
- R² und R³: unabhängig voneinander für Fluor, Chlor, Nitro, Methyl, Methoxy, Methylthio, Methylsulfinyl oder Methylsulfonyl stehen.

Eine weitere ganz besonders bevorzugt verwendbare Gruppe von Acetophenon-Derivaten sind Stoffe der Formel (II), in denen
- R², R⁴ und R⁵: für Wasserstoff stehen und
- R¹ und R³: unabhängig voneinander für Fluor, Chlor, Nitro, Methyl, Methoxy, Methylthio, Methylsulfinyl oder Methylsulfonyl stehen.

Eine weitere ganz besonders bevorzugt verwendbare Gruppe von Acetophenon-Derivaten sind Stoffe der Formel (II), in denen
- R², R³ und R⁴: für Wasserstoff stehen und
- R¹ und R⁵: unabhängig voneinander für Fluor, Chlor, Nitro, Methyl, Methoxy, Methylthio, Methylsulfinyl oder Methylsulfonyl stehen.

Eine weitere ganz besonders bevorzugt verwendbare Gruppe von Acetophenon-Derivaten sind Stoffe der Formel (II), in denen
- R¹, R³ und R⁵: für Wasserstoff stehen und
- R² und R⁴: unabhängig voneinander für Fluor, Chlor, Nitro, Methyl, Methoxy, Methylthio, Methylsulfinyl oder Methylsulfonyl stehen.

Eine weitere ganz besonders bevorzugt verwendbare Gruppe von Acetophenon-Derivaten sind Stoffe der Formel (II), in denen
- R², R³, R⁴ und R⁵: für Wasserstoff stehen und
- R¹: für Fluor, Chlor, Nitro, Methyl, Methoxy, Methylthio, Methylsulfinyl oder Methylsulfonyl steht.

Eine weitere ganz besonders bevorzugt verwendbare Gruppe von Acetophenon-Derivaten sind Stoffe der Formel (II), in denen
- R¹, R³, R⁴ und R⁵: für Wasserstoff stehen und
- R²: für Fluor, Chlor, Nitro, Methyl, Methoxy, Methylthio, Methylsulfinyl oder Methylsulfonyl steht.

Eine weitere ganz besonders bevorzugt verwendbare Gruppe von Acetophenon-Derivaten sind Stoffe der Formel (II), in denen
- R¹, R², R⁴ und R⁵: für Wasserstoff stehen und
- R³: für Fluor, Chlor, Nitro, Methyl, Methoxy, Methylthio, Methylsulfinyl oder Methylsulfonyl steht.

Die Acetophenon-Derivate der Formel (II) sind bekannt oder lassen sich nach bekannten Methoden herstellen.

Als Verdünnungsmittel zur Durchführung der ersten Stufe des erfindungsgemäßen Verfahrens kommen alle inerten organischen Lösungsmittel in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin, halogenierte Kohlenwasserstoffe, wie Chlorbenzol, Dichlorbenzol, Dichlormethan, Chloroform, Tetrachlormethan, Dichlorethan oder Trichlorethan; Nitrile, wie n- oder i-Butyronitril oder Benzonitril.

Als Katalysatoren kommen bei der Durchführung der ersten Stufe des erfindungsgemäßen Verfahrens alle für derartige Umsetzungen üblichen sauren Reaktionsbeschleuniger in Frage. Vorzugsweise verwendbar sind anorganische oder organische Protonen- oder Lewissäuren und auch polymere Säuren, wie z.B. Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Titantetrachlorid, Tetrabutylorthotitanat, Zinkchlorid, Eisen-III-chlorid, Antimonpentachlorid, saure Ionenaustauscher, saure Tonerden und saures Kieselgel.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen 20°C und 200°C, vorzugsweise zwischen 20°C und 150°C.

Sowohl bei der Durchführung der ersten als auch der zweiten und dritten Stufe des erfindungsgemäßen Verfahrens arbeitet man im allgemeinen unter Atmosphärendruck. Es ist aber auch möglich, unter erhöhtem oder vermindertem Druck, im allgemeinen zwischen 0,1 und 10 bar, zu arbeiten.

Bei der Durchführung der ersten Stufe des erfindungsgemäßen Verfahrens setzt man auf 1 Mol an optisch aktivem Phenethylamin der Formel (I*) im allgemeinen 0,5 bis 5 Mol, vorzugsweise 0,8 bis 2 Mol an Acetophenon-Derivat der Formel (II) sowie gegebenenfalls eine geringe Menge, im allgemeinen zwischen 10⁻³ und 10⁻⁵ Mol an Katalysator ein. Im einzelnen verfährt man im allgemeinen in der Weise, daß man die Ausgangssubstanzen in einem mit Wasser wenig mischbaren Verdünnungsmittel löst, danach mit Katalysator versetzt und zum Sieden erhitzt. Das bei der Reaktion entstehende Wasser wird im allgemeinen azeotrop abdestilliert und gegebenenfalls in einem Wasserabscheider aufgefangen. Die Isolierung der Reaktionsprodukte erfolgt im allgemeinen in der Weise, daß man das Reaktionsgemisch zunächst filtriert und dann unter vermindertem Druck einengt. Das dabei erhaltene Produkt ist im allgemeinen für weitere Umsetzungen ausreichend rein. Es kann aber auch durch übliche Reinigungsmethoden, wie z.B. durch Chromatographie oder Umkristallisation, von noch vorhandenen Verunreinigungen befreit werden.

Als Racemisierungsreagenzien kommen bei der Durchführung der zweiten Stufe des erfindungsgemäßen Verfahrens alle üblichen Metallhydroxide in Betracht, die einen Wassergehalt zwischen 0,1 und 50 Gew.-%, vorzugsweise zwischen 0,1 und 30 Gew.-% aufweisen. Vorzugsweise verwendbar sind Alkalimetall- oder Erdalkalimetallhydroxide, wie Natriumhydroxid, Kaliumhydroxid, Calciumhydroxid oder Magnesiumhydroxid. Der Wassergehalt kann gegebenenfalls durch Zugabe von Wasser eingestellt werden.

Die Reaktionstemperaturen können auch bei der Durchführung der zweiten Stufe des erfindungsgemäßen Verfahrens in einem größeren Bereich variiert werden. Im allgemeinen arbeitet man bei Temperaturen zwischen 50°C und 250°C, vorzugsweise zwischen 80°C und 180°C.

Bei der Durchführung der zweiten Stufe des erfindungsgemäßen Verfahrens arbeitet man im allgemeinen unter Schutzgasatmosphäre Als Schutzgase kommen dabei vorzugsweise inerte Gase, wie Stickstoff oder Argon, in Frage.

Bei der Durchführung der zweiten Stufe des erfindungsgemäßen Verfahrens setzt man auf 1 Mol an optisch aktiver Schiffscher Base der Formel (III*) im allgemeinen 1 bis 10 Mol, vorzugsweise 1 bis 5 Mol Metallhydroxid ein und fügt gegebenenfalls zusätzliches Wasser hinzu. Im einzelnen verfährt man im allgemeinen in der Weise, daß man das Reaktionsgemisch gegebenenfalls unter Schutzgasatmosphäre auf die gewünschte Temperatur erhitzt, danach abkühlt und in üblicher Weise aufarbeitet. In einer bevorzugten Variante geht man jedoch so vor, daß man das abgekühlte Reaktionsgemisch mit einer so großen Menge an Säure versetzt, daß ein stark saures Gemisch entsteht, welches direkt zur weiteren Durchführung der dritten Stufe verwendet werden kann.

Als Säuren zur Spaltung der Schiffschen Basen der Formel (III) kommen bei der Durchführung der dritten Stufe des erfindungsgemäßen Verfahrens alle üblichen anorganischen oder organischen Säuren oder auch saure Polymere, jeweils in Gegenwart von Wasser, in Frage. Vorzugsweise verwendbar sind Chlorwasserstoffsäure, Schwefelsäure, Phosphorsäure, Ameisensäure, Essigsäure, Trifluoressigsäure, Methansulfonsäure, Trifluormethansulfonsäure, Toluolsulfonsäure oder saure Ionenaustauscher.

Die Reaktionstemperaturen können auch bei der Durchführung der dritten Stufe des erfindungsgemäßen Verfahrens in einem größeren Bereich variiert werden. Im allgemeinen arbeitet man bei Temperaturen zwischen 20°C und 150°C, vorzugsweise zwischen 20°C und 120°C.

Bei der Durchführung der dritten Stufe des erfindungsgemäßen Verfahrens setzt man auf 1 Mol an Schiffscher Base der Formel (III) im allgemeinen einen Überschuß an Säure sowie Wasser ein. Vorzugsweise geht man so vor, daß man das bei der Durchführung der zweiten Stufe anfallende Gemisch ohne vorherige Isolierung der racemischen Schiffschen Base mit wäßriger Säure oder mit Wasser und Säure versetzt und das entstehende Gemisch bis zur Beendigung der Umsetzung auf die gewünschte Temperatur erhitzt. Die Aufarbeitung erfolgt nach üblichen Methoden. Im allgemeinen geht man so vor, daß man das Reaktionsgemisch mit einem mit Wasser wenig mischbaren Solvens extrahiert, die organische Phase trocknet und dann unter vermindertem Druck einengt. Das dabei verbleibende Acetophenon-Derivat kann gegebenenfalls nach vorheriger Reinigung erneut zur Durchführung der ersten Stufe des erfindungsgemäßen Verfahrens eingesetzt werden. Die nach der Extraktion verbleibende wäßrige Phase wird im allgemeinen in der Weise aufgearbeitet, daß man zunächst durch Zugabe von wäßriger Lauge basisch stellt, das entstehende Gemisch mit einem mit Wasser wenig mischbaren organischen Solvens extrahiert, die organische Phase trocknet und unter vermindertem Druck einengt. Das anfallende Produkt kann nach üblichen Methoden, wie Destillation, Kristallisation oder Chromatographie, von noch vorhandenen Verunreinigungen befreit werden.

Die nach dem erfindungsgemäßen Verfahren erhältlichen racemischen Phenethylamine der Formel (I) können entweder direkt oder nach vorheriger Racematspaltung als Zwischenprodukte für weitere Synthesen eingesetzt werden. Vor allem (R)-Phenethylmine der Formel (I) sind wertvolle Zwischenprodukte zur Herstellung von Pharmazeutika oder von Wirkstoffen mit insektiziden, fungiziden oder herbiziden Eigenschaften (vgl. EP-A 0 519 211, EP-A 0 453 137, EP-A 0 283 879, EP-A 0 264 217 und EP-A 0 341 475).

So läßt sich z.B. aus racemischem l-(4-Chlor-phenyl)-ethylamin das (R)-Enantiomere gewinnen, indem man eine Lösung des Racemates in Ethanol und eine Lösung von (S)-(-)-N-Phenylcarbamat-milchsäure in Ethanol bei Temperaturen zwischen 60°C und 70°C zusammengibt, den dabei entstehenden Kristallbrei absaugt und mit wäßriger Natronlauge in Gegenwart von Methylenchlorid behandelt (vgl. EP-A 0 341 475) Aus dem (R)-1-(4-Chlor-phenyl)-ethylamin läßt sich das fungizid wirksame Diastereomerengemisch aus N-(R)-[1-(4-Chlor-phenyl)-ethyl]-(IR)-2,2-dichlor-1-ethyl-3t-methyl-1r-cyclopropan-carbonsäureamid und N-(R)-[1-(4-Chlor-phenyl)-ethyl]-(1S)-2,2-dichlor-1-ethyl-3t-methyl-1r-cyclopropan-Carbonsäureamid der Formeln 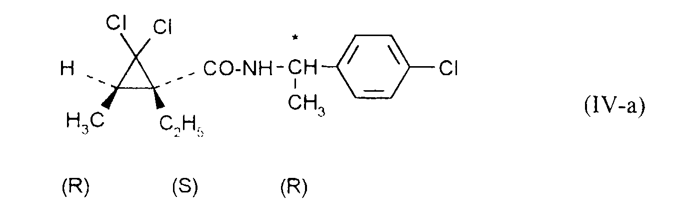 und 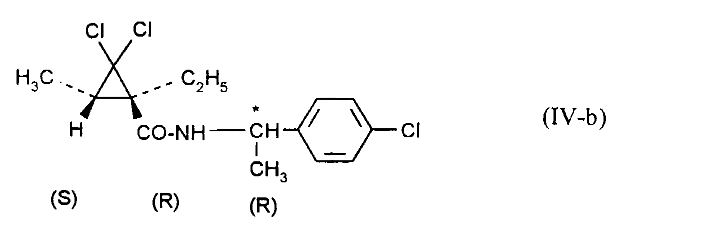 herstellen, indem man ein 1:1-Gemisch aus (1R)-2,2-Dichlor-1-ethyl-3t-methyl-1r-cyclopropan-carbonsäurechlorid und (1S)-2,2-Dichlor-1-ethyl-3t-methyl-1r-cyclopropan-carbonsäurechlorid der Formeln 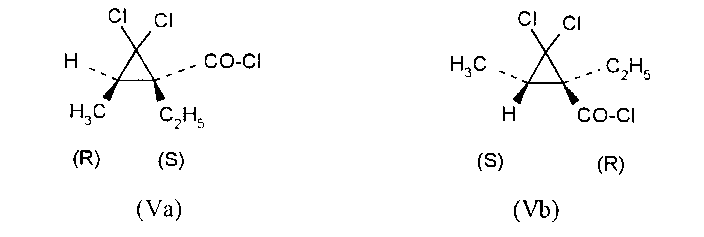 mit R-1-(4-Chlor-phenyl)-ethylamin der Formel 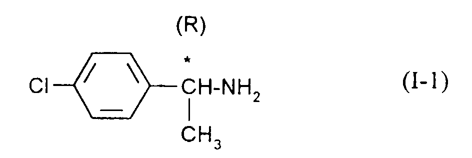 in Gegenwart eines Verdünnungsmittels, wie Methylchlorid, und in Gegenwart eines Säurebindemittels, wie Triethylamin, umsetzt.

Die Durchführung des erfindungsgemäßen Verfahrens wird durch die folgenden Beispiele veranschaulicht.

### Herstellungsbeispiele

### Beispiel 1

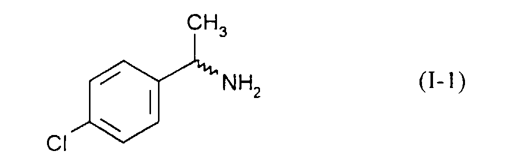

### Erste Stufe:

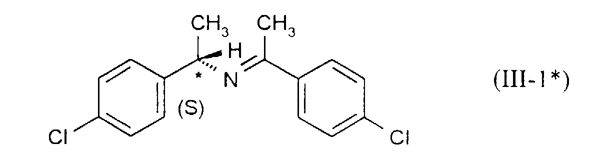

Eine Lösung von 116 g (0,72 Mol) (S)-l-(4-Chlor-phenyl)-ethylamin (65 % ee) und 114,5 g (0,727 Mol) 4-Chloracetophenon in 500 ml Toluol wird bei Raumtemperatur unter Rühren mit 8 g Tetrabutyl-ortho-titanat versetzt und dann 6 Stunden am Wasserabscheider unter Rückfluß erhitzt. Die Reaktionsmischung wird filtriert, und das Filtrat wird unter vermindertem Druck eingeengt. Man erhält 191,3 g (91 % der Theorie) (S)-[1-(4-Chlorphenyl)-ethyl]-[1-(4-chlorphenyl)-ethyliden]-amin.
¹H-NMR (CDCl₃ ,TMS, 300 MHz): δ = 1,48 (d, 3H), 2,24 (s, 3H), 4,72 (q, 1H), 7,18 - 7,45 (m, 8H) ppm

### Zweite und dritte Stufe:

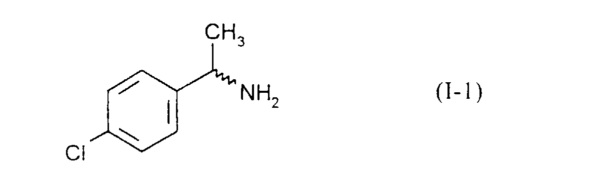

Ein Gemisch aus 89 g (0,3 Mol) (S)-[1-(4-Chlorphenyl)-ethyl]-[1-(4-chlorphenyl)-ethyliden]-amin (65 % ee) und 39,6 g (0,7 Mol) Kaliumhydroxid mit einem Wassergehalt von 15 Gew.-% wird unter Rühren 16 Stunden lang auf 130 bis 160°C erhitzt. Danach wird das Reaktionsgemisch abgekühlt, mit 100 ml 2-normaler wäßriger Schwefelsäure versetzt und 2 Stunden unter Rückfluß erhitzt. Das Gemisch wird auf Raumtemperatur abgekühlt und dreimal mit Dichlormethan extrahiert.

Die wäßrige Phase wird unter Kühlung mit konzentrierter, wäßriger Natronlauge alkalisch gestellt. Das dabei entstehende Gemisch wird dreimal mit Dichlormethan extrahiert. Die vereinigten organischen Phasen werden über Natriumsulfat getrocknet und dann unter verminderten, Druck eingeengt. Man erhält 39,4 g eines Produktes, das gemäß gaschromatographischer Analyse zu 96,3 % aus racemischem 1-(4-Chlor-phenyl)-ethylamin besteht. Die Ausbeute errechnet sich danach zu 84 % der Theorie.

### Beispiel 2

### Zweite und dritte Stufe:

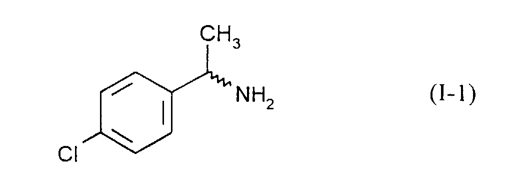

Ein Gemisch aus 89 g (0,3 Mol) (S)-[1-(4-Chlorphenyl)-ethyl]-[1-(4-chlorphenyl)-ethyliden]-amin (65 % ee) und 39,6 g (0,7 Mol) Kaliumhydroxid mit einem Wassergehalt von 15 Gew-% wird mit 6,1 g Wasser versetzt und unter Rühren 16 Stunden lang auf 130 bis 160°C erhitzt. Danach wird das Reaktionsgemisch abgekühlt, mit 100 ml 2-normaler wäßriger Schwefelsäure versetzt und 2 Stunden unter Rückfluß erhitzt. Das Gemisch wird auf Raumtemperatur abgekühlt und dreimal mit Dichlormethan extrahiert.

Die wäßrige Phase wird unter Kühlung mit konzentrierter, wäßriger Natronlauge alkalisch gestellt. Das dabei entstehende Gemisch wird dreimal mit Dichlormethan extrahiert Die vereinigten organischen Phasen werden über Natriumsulfat getrocknet und dann unter vermindertem, Druck eingeengt. Man erhält 38,1 g eines Produktes, das gemäß gaschromatographischer Analyse zu 95,7 % aus racemischem 1-(4-Chlor-phenyl)-ethylamin besteht. Die Ausbeute errechnet sich danach zu 82 % der Theorie.

### Beispiel 3

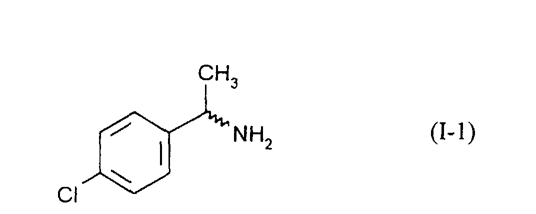

### Erste Stufe:

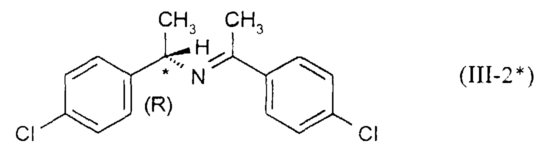

Eine Lösung von 52 g (0,35 Mol) (R)-1-(4-Chlor-phenyl)-ethylamin (97 % ee) und 51,7 g (0,353 Mol) 4-Chloracetophenon in 300 ml Toluol wird bei Raumtemperatur unter Rühren mit 3 g Tetrabutyl-ortho-Titanat versetzt und dann 2 Stunden am Wasserabscheider unter Rückfluß erhitzt. Man fügt erneut 2 g Tetrabutyl-ortho-Titanat hinzu und erhitzt weitere 6 Stunden unter Rückfluß. Anschließend wird noch einmal mit 2 g Tetrabutyl-ortho-Titanat versetzt und dann 3 Stunden unter Rückfluß erhitzt. Danach wird das Reaktionsgemisch filtriert. Das Filtrat wird unter vermindertem Druck eingeengt. Man erhält 67,3 g (66 % der Theorie) (R)-[1-(4-Chlorphenyl)-ethyl]-[1-(4-chlorphenyl)-ethyliden]-amin
¹H-NMR (CDCl₃ ,TMS, 300 MHz): δ = 1,48 (d, 3H), 2,24 (s, 3H), 4,72 (q, 1H), 7,18 - 7,45 (m, 8H) ppm

### Zweite und dritte Stufe:

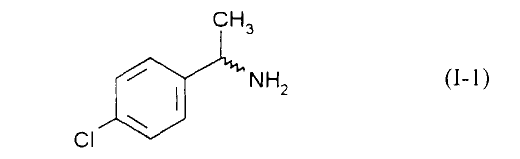

Ein Gemisch aus 9,6 g (0,026 Mol) (R)-[1-(4-Chlorphenyl)-ethyl]-[1-(4-chlorphenyl)-ethyliden]-amin (97 % ee) und 4,22 g (0,075 Mol) Kaliumhydroxid mit einem Wassergehalt von 15 Gew.-% wird unter Rühren 20 Stunden lang auf 150 bis 160°C erhitzt. Danach wird das Reaktionsgemisch abgekühlt, mit 35 ml 2-normaler wäßriger Schwefelsäure versetzt und 4 Stunden unter Rückfluß erhitzt. Das Gemisch wird auf Raumtemperatur abgekühlt und dreimal mit Dichlormethan extrahiert.

Die wäßrige Phase wird unter Kühlung mit konzentrierter, wäßriger Natronlauge alkalisch gestellt. Das dabei entstehende Gemisch wird dreimal mit Dichlormethan extrahiert. Die vereinigten organischen Phasen werden über Natriumsulfat getrocknet und dann unter vermindertem Druck eingeengt. Man erhält 3,7 g eines Produktes, das gemäß gaschromatographischer Analyse zu 97 % aus racemischem 1-(4-Chlor-phenyl)-ethylamin besteht. Die Ausbeute errechnet sich danach zu 87 % der Theorie.

### Vergleichsbeispiel A

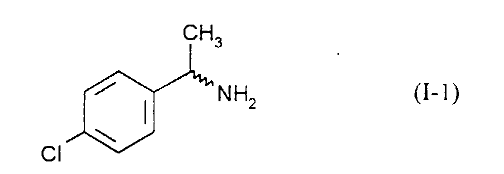

### Erste Stufe:

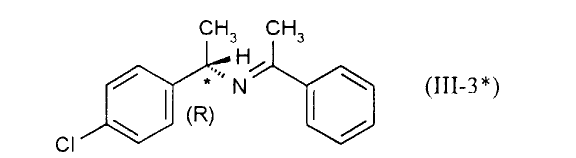

Eine Lösung von 20 g (0,128 Mol) (R)-1-(4-Chlor-phenyl)-ethylamin (97 % ee) und 15,6 g (0,13 Mol) Acetophenon in 70 ml Toluol wird bei Raumtemperatur unter Rühren mit 0,3 g Zinkchlorid versetzt und dann 74 Stunden am Wasserabscheider unter Rückfluß erhitzt. Danach wird das Reaktionsgemisch filtriert. Das Filtrat wird unter vermindertem Druck eingeengt. Man erhält 15,56 g (47 % der Theorie) an (R)-[1-(4-Chlorphenyl)-ethyl]-[1-phenyl-ethyliden]-amin.

### Zweite und dritte Stufe:

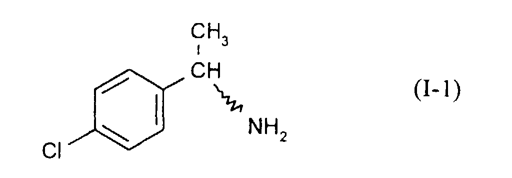

Ein Gemisch aus 6,2 g (R)-[1-(4-Chlorphenyl)-ethyl]-[1-phenyl-ethyliden]-amin (97 % ee) und 0,75 g Kalium-tert-butanolat wird unter Rühren unter Argonatmosphäre 6 Stunden auf 110°C erhitzt. Danach wird das Reaktionsgemisch auf Raumtemperatur abgekühlt, mit 17 ml 2-normaler wäßriger Schwefelsäure versetzt und 1,5 Stunden unter Rückfluß erhitzt. Das Gemisch wird auf Raumtemperatur abgekühlt und zweimal mit Dichlormethan extrahiert.

Die wäßrige Phase wird unter Kühlung mit 10 %iger, wäßriger Natronlauge alkalisch gestellt. Das dabei entstehende Gemisch wird dreimal mit Dichlormethan extrahiert. Die vereinigten organischen Phasen werden über Natriumsulfat getrocknet und dann unter vermindertem Druck eingeengt. Man erhält 2,1 g eines Produktes, das gemäß gaschromatographischer Analyse zu 61,64 % aus racemischem 1-(4-Chlor-phenyl)-ethylamin besteht. Die Ausbeute errechnet sich danach zu 36 % der Theorie.

### Vergleichsbeispiel B

### Zweite und dritte Stufe:

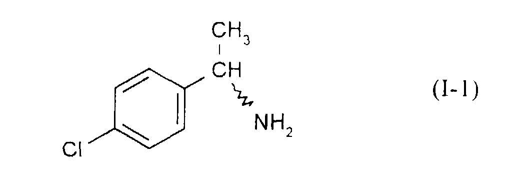

Ein Gemisch aus 3,54 g (0,01Mol) (S)-[1-(4-Chlorphenyl)-ethyl]-[1-(4-chlorphenyl)-ethyliden]-amin (63,5 % ee) und 0,66 g (0,01 Mol) Kaliumhydroxid mit einem Wassergehalt von 15 Gew.-% wird mit 0,2 g Wasser versetzt und unter Rühren 20 Stunden auf 130°C erhitzt. Danach wird das Reaktionsgemisch abgekühlt, mit 40 ml 2-normaler wäßriger Schwefelsäure versetzt und 3 Stunden unter Rückfluß erhitzt. Das Gemisch wird auf Raumtemperatur abgekühlt und zweimal mit Dichlormethan extrahiert.

Die wäßrige Phase wird unter Kühlung mit konzentrierter, wäßriger Natronlauge alkalisch gestellt. Das dabei entstehende Gemisch wird dreimal mit Dichlormethan extrahiert. Die vereinigten organischen Phasen werden über Natriumsulfat getrocknet und dann unter vermindertem, Druck eingeengt Man erhält 1,45 g eines Produktes, das gemäß gaschromatographischer Analyse zu 98,3 % aus (S)-1-(4-Chlor-phenyl)-ethylamin (ee-Wert 64,15 %) besteht.

## Patentansprüche

1. Verfahren zur Herstellung von racemischen Phenethylaminen der Formel 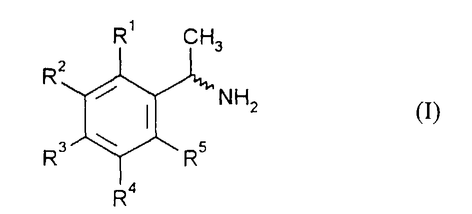 in welcher
R¹, R², R³, R⁴ und R⁵ unabhängig voneinander für Wasserstoff, Halogen, Cyano, Nitro, Alkyl, Alkoxy, Alkylthio, Alkylsulfinyl, Alkylsulfonyl, Dialkylamino, Halogenalkyl, Halogenalkoxy, Halogenalkylthio, Halogenalkylsulfinyl oder Halogenalkylsulfonyl stehen,
dadurch gekennzeichnet, daß man
a) in einer ersten Stufe optisch aktive Phenethylamine der Formel 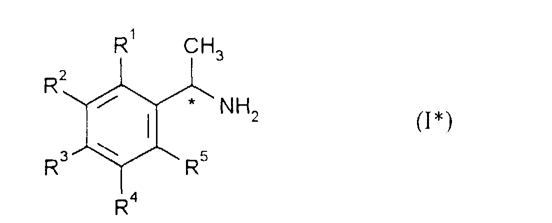 in welcher
R¹, R², R³, R⁴ und R⁵ die oben angegebenen Bedeutungen haben,
mit Acetophenon-Derivaten der Formel 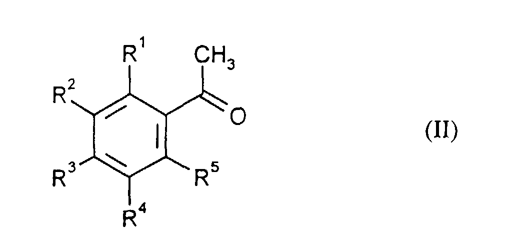 in welcher
R¹, R², R³, R⁴ und R⁵ die oben angegebenen Bedeutungen haben,
wobei das eingesetzte optisch aktive Phenethylamin und das eingesetzte Acetophenon-Derivat im Phenylteil jeweils identisch substituiert sind,
gegebenenfalls in Gegenwart eines Verdünnungsmittels und gegebenenfalls in Gegenwart eines Katalysators umsetzt,
b) dann in einer zweiten Stufe die entstandenen optisch aktiven Schiffschen Basen der Formel 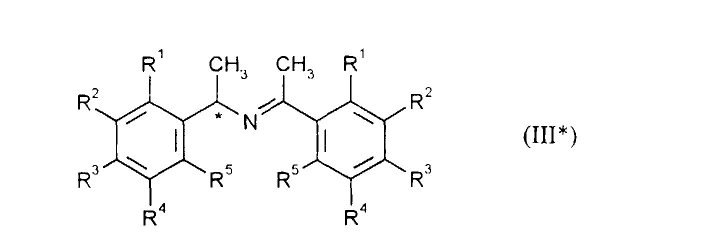 in welcher
R¹, R², R³, R⁴ und R⁵ die oben angegebenen Bedeutungen haben,
mit Metallhydroxiden, die einen Wassergehalt zwischen 0,1 und 50 Gew.-% aufweisen, gegebenenfalls unter Schutzgasatmosphäre umsetzt und
c) in einer dritten Stufe die erhaltenen racemischen Schiffschen Basen der Formel 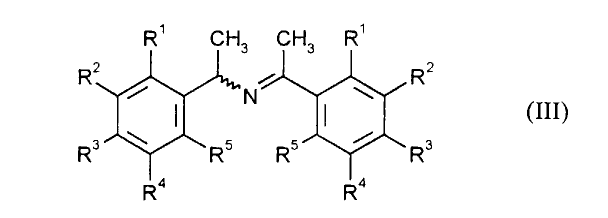 in welcher
R¹, R², R³, R⁴ und R⁵ die oben angegebenen Bedeutungen haben,
mit Säuren in Gegenwart von Wasser umsetzt.

2. Verfahren gemäß Anspruch 1, dadurch gekennzeichnet, daß man als Ausgangsstoffe optisch aktive Phenethylamine der Formel (I*) einsetzt, in denen
R¹, R², R³, R⁴ und R⁵ unabhängig voneinander für Wasserstoff, Fluor, Chlor, Brom, Cyano, Nitro, Alkyl mit 1 bis 4 Kohlenstoffatomen, Alkoxy mit 1 oder 2 Kohlenstoffatomen, Alkylthio mit 1 oder 2 Kohlenstoffatomen, Alkylsulfinyl mit 1 oder 2 Kohlenstoffatomen, Alkylsulfonyl mit 1 oder 2 Kohlenstoffatomen, Dialkylamino mit jeweils 1 oder 2 Kohlenstoffatomen in den Alkylketten, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Halogenalkylsulfinyl mit 1 oder 2 Kohlenstoffatomen und 1 bis 5 Halogenatomen oder Halogenalkylsulfonyl mit 1 oder 2 Kohlenstoffatomen und 1 bis 5 Halogenatomen stehen.

3. Verfahren gemäß Anspruch 1, dadurch gekennzeichnet, daß man bei der Durchführung der ersten Stufe Acetophenon-Derivate der Formel (II) als Reaktionskomponenten einsetzt, in denen
R¹, R², R³, R⁴ und R⁵ unabhängig voneinander für Wasserstoff, Fluor, Chlor, Brom, Cyano, Nitro, Alkyl mit 1 bis 4 Kohlenstoffatomen, Alkoxy mit 1 oder 2 Kohlenstoffatomen, Alkylthio mit 1 oder 2 Kohlenstoffatomen, Alkylsulfinyl mit 1 oder 2 Kohlenstoffatomen, Alkylsulfonyl mit 1 oder 2 Kohlenstoffatomen, Dialkylamino mit jeweils 1 oder 2 Kohlenstoffatomen in den Alkylketten, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Halogenalkylsulfinyl mit 1 oder 2 Kohlenstoffatomen und 1 bis 5 Halogenatomen oder Halogenalkylsulfonyl mit 1 oder 2 Kohlenstoffatomen und 1 bis 5 Halogenatomen stehen.

4. Verfahren gemäß Anspruch 1, dadurch gekennzeichnet, daß man bei der Durchführung der ersten Stufe (S)-1-(4-Chlor-phenyl)-ethylamin mit 4-Chloracetophenon umsetzt.

5. Verfahren gemäß Anspruch 1, dadurch gekennzeichnet, daß man bei der Durchführung der ersten Stufe in Gegenwart eines sauren Katalysators arbeitet.

6. Verfahren gemäß Anspruch 1, dadurch gekennzeichnet, daß man bei der Durchführung der zweiten Stufe Alkalimetall- oder Erdalkalimetallhydroxide mit einem Wassergehalt zwischen 0,1 und 30 Gew.-% als Racemisierungsreagenzien einsetzt.

7. Verfahren gemäß Anspruch 1, dadurch gekennzeichnet, daß man als Säuren bei der Durchführung der dritten Stufe anorganische oder organische Säuren oder saure Polymere, jeweils in Gegenwart von Wasser, einsetzt.

8. Verfahren gemäß Anspruch 1, dadurch gekennzeichnet, daß man bei der Durchführung der ersten Stufe bei Temperaturen zwischen 20°C und 200°C, bei der Durchführung der zweiten Stufe bei Temperaturen zwischen 50°C und 250°C und bei der Durchführung der dritten Stufe bei Temperaturen zwischen 20°C und 150°C arbeitet.

## Claims

1. Process for the preparation of racemic phenethylamines of the formula 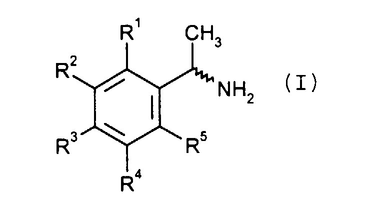 in which
R¹, R², R³, R⁴ and R⁵ independently of one another are hydrogen, halogen, cyano, nitro, alkyl, alkoxy, alkylthio, alkylsulphinyl, alkylsulphonyl, dialkylamino, halogenoalkyl, halogenoalkoxy, halogenoalkylthio, halogenoalkylsulphinyl or halogenoalkylsulphonyl,
characterized in that
a) in a first stage, optically active phenethylamines of the formula 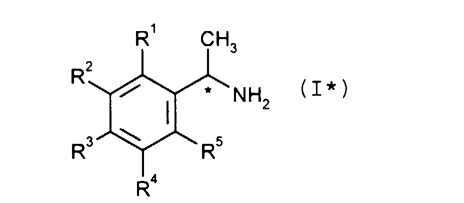 in which
R¹, R², R³, R⁴ and R⁵ are as defined above,
are reacted with acetophenone derivatives of the formula 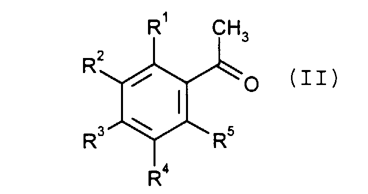 in which
R¹, R², R³, R⁴ and R⁵ are as defined above,
the optically active phenethylamine used and the acetophenone derivative used each being identically substituted in the phenyl moiety,
optionally in the presence of a diluent and optionally in the presence of a catalyst,
b) then in a second stage, the resulting optically active Schiff bases of the formula 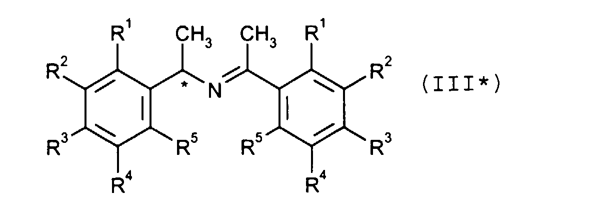 in which
R¹, R², R³, R⁴ and R⁵ are as defined above,
are reacted with metal hydroxides having a water content of between 0.1 and 50% by weight, optionally under a protective-gas atmosphere, and
c) in a third stage, the resulting racemic Schiff bases of the formula 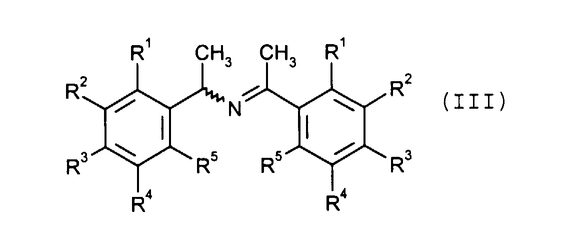 in which
R¹, R², R³, R⁴ and R⁵ are as defined above,
are reacted with acids in the presence of water.

2. Process according to Claim 1, characterized in that the starting materials used are optically active phenethylamines of the formula (I*) in which
R¹, R², R³, R⁴ and R⁵ independently of one another are hydrogen, fluorine, chlorine, bromine, cyano, nitro, alkyl having from 1 to 4 carbon atoms, alkoxy having 1 or 2 carbon atoms, alkylthio having 1 or 2 carbon atoms, alkylsulphinyl having 1 or 2 carbon atoms, alkylsulphonyl having 1 or 2 carbon atoms, dialkylamino having in each case 1 or 2 carbon atoms in the alkyl chains, halogenoalkyl having 1 or 2 carbon atoms and from 1 to 5 halogen atoms, halogenoalkoxy having 1 or 2 carbon atoms and from 1 to 5 halogen atoms, halogenoalkylthio having 1 or 2 carbon atoms and from 1 to 5 halogen atoms, halogenoalkylsulphinyl having 1 or 2 carbon atoms and from 1 to 5 halogen atoms or halogenoalkylsulphonyl having 1 or 2 carbon atoms and from 1 to 5 halogen atoms.

3. Process according to Claim 1, characterized in that the first stage is carried out using, as reaction components, acetophenone derivatives of the formula (II) in which
R¹, R², R³, R⁴ and R⁵ independently of one another are hydrogen, fluorine, chlorine, bromine, cyano, nitro, alkyl having from 1 to 4 carbon atoms, alkoxy having 1 or 2 carbon atoms, alkylthio having 1 or 2 carbon atoms, alkylsulphinyl having 1 or 2 carbon atoms, alkylsulphonyl having 1 or 2 carbon atoms, dialkylamino having in each case 1 or 2 carbon atoms in the alkyl chains, halogenoalkyl having 1 or 2 carbon atoms and from 1 to 5 halogen atoms, halogenoalkoxy having 1 or 2 carbon atoms and from 1 to 5 halogen atoms, halogenoalkylthio having 1 or 2 carbon atoms and from 1 to 5 halogen atoms, halogenoalkylsulphinyl having 1 or 2 carbon atoms and from 1 to 5 halogen atoms or halogenoalkylsulphonyl having 1 or 2 carbon atoms and from 1 to 5 halogen atoms.

4. Process according to Claim 1, characterized in that, in the first stage, (S)-1-(4-chloro-phenyl)-ethylamine is reacted with 4-chloroacetophenone .

5. Process according to Claim 1, characterized in that the first stage is carried out in the presence of an acidic catalyst.

6. Process according to Claim 1, characterized in that, in the second stage, alkali metal or alkaline earth metal hydroxides having a water content of between 0.1 and 30% by weight are used as racemization reagents.

7. Process according to Claim 1, characterized in that the acids used in the third stage are inorganic or organic acids or acidic polymers, each in the presence of water.

8. Process according to Claim 1, characterized in that the first stage is carried out at temperatures between 20°C and 200°C, the second stage is carried out at temperatures between 50°C and 250°C, and the third stage is carried out at temperatures between 20°C and 150°C.

## Revendications

1. Procédé de production de phénéthylamines racémiques de formule 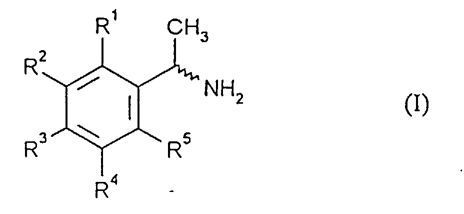 dans laquelle
R¹, R², R³, R⁴ et R⁵ représentent, indépendamment les uns des autres, l'hydrogène, un halogène, un groupe cyano, nitro, alkyle, alkoxy, alkylthio, alkylsulfinyle, alkylsulfonyle, dialkylamino, halogénalkyle, halogénalkoxy, halogénalkylthio, halogénalkylsulfinyle ou halogénalkylsulfonyle,
caractérisé en ce que
a) on fait réagir dans une première étape des phénéthylamines optiquement actives de formule 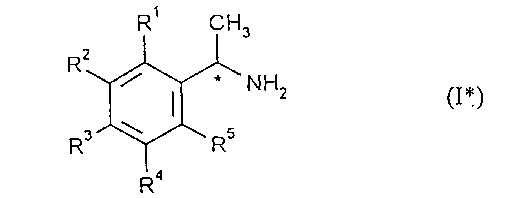 dans laquelle
R¹, R², R³, R⁴ et R⁵ ont les définitions indiquées ci-dessus,
avec des dérivés d'acétophénone de formule 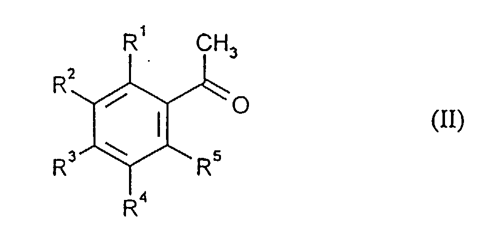 dans laquelle
R¹, R², R³, R⁴ et R⁵ ont les définitions indiquées ci-dessus,
la phénéthylamine optiquement active et le dérivé d'acétophénone utilisés étant substitués chacun de façon identique dans la partie phényle,
le cas échéant en présence d'un diluant et en la présence éventuelle d'un catalyseur,
b) on fait ensuite réagir dans une deuxième étape les bases de Schiff optiquement actives produites de formule 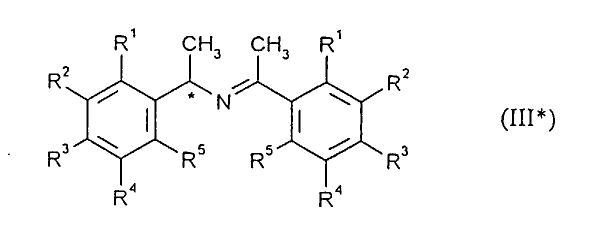 dans laquelle
R¹, R², R³, R⁴ et R⁵ ont les définitions indiquées ci-dessus,
avec des hydroxydes de métaux qui présentent une teneur en eau comprise entre 0,1 et 50 % en poids, éventuellement sous atmosphère de gaz protecteur, et
c) on fait réagir dans une troisième étape les bases de Schiff racémiques obtenues de formule 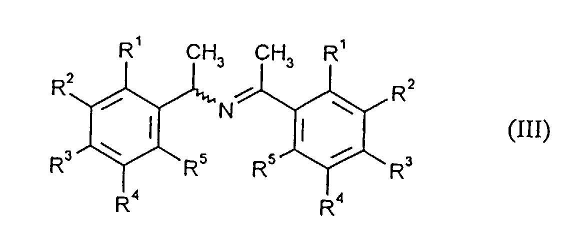 dans laquelle
R¹, R², R³, R⁴ et R⁵ ont les définitions indiquées ci-dessus,
avec des acides en présence d'eau.

2. Procédé suivant la revendication 1, caractérisé en ce qu'on utilise comme composés de départ des phénéthylamines optiquement actives de formule (I*), dans lesquelles
R¹, R², R³, R⁴ et R⁵ représentent, indépendamment les uns des autres, l'hydrogène, le fluor, le chlore, le brome, un groupe cyano, nitro, alkyle ayant 1 à 4 atomes de carbone, alkoxy ayant 1 ou 2 atomes de carbone, alkylthio ayant 1 ou 2 atomes de carbone, alkylsulfinyle ayant 1 ou 2 atomes de carbone, alkylsulfonyle ayant 1 ou 2 atomes de carbone, dialkylamino ayant 1 ou 2 atomes de carbone dans chacune des chaînes alkyle, halogénalkyle ayant 1 ou 2 atomes de carbone et 1 à 5 atomes d'halogènes, halogénalkoxy ayant 1 ou 2 atomes de carbone et 1 à 5 atomes d'halogènes, halogénalkylthio ayant 1 ou 2 atomes de carbone et 1 à 5 atomes d'halogènes, halogénalkylsulfinyle ayant 1 ou 2 atomes de carbone et 1 à 5 atomes d'halogènes ou halogénalkylsulfonyle ayant 1 ou 2 atomes de carbone et 1 à 5 atomes d'halogènes.

3. Procédé suivant la revendication 1, caractérisé en ce qu'on utilise dans la conduite de la première étape des dérivés d'acétophénone de formule (II) comme composants réactionnels, dans lesquels
R¹, R², R³, R⁴ et R⁵ représentent, indépendamment les uns des autres, l'hydrogène, le fluor, le chlore, le brome, un groupe cyano, nitro, alkyle ayant 1 à 4 atomes de carbone, alkoxy ayant 1 ou 2 atomes de carbone, alkylthio ayant 1 ou 2 atomes de carbone, alkylsulfinyle ayant 1 ou 2 atomes de carbone, alkylsulfonyle ayant 1 ou 2 atomes de carbone, dialkylamino ayant 1 ou 2 atomes de carbone dans chacune des chaînes alkyle, halogénalkyle ayant 1 ou 2 atomes de carbone et 1 à 5 atomes d'halogènes, halogénalkoxy ayant 1 ou 2 atomes de carbone et 1 à 5 atomes d'halogènes, halogénalkylthio ayant 1 ou 2 atomes de carbone et 1 à 5 atomes d'halogènes, halogénalkylsulfinyle ayant 1 ou 2 atomes de carbone et 1 à 5 atomes d'halogènes ou halogénalkylsulfonyle ayant 1 ou 2 atomes de carbone et 1 à 5 atomes d'halogènes.

4. Procédé suivant la revendication 1, caractérisé en ce qu'on fait réagir, dans la conduite de la première étape, la (S)-1-(4-chlorophényl)-éthylamine avec la 4-chloracétophénone.

5. Procédé suivant la revendication 1, caractérisé en ce qu'on opère dans la conduite de la première étape en présence d'un catalyseur acide.

6. Procédé suivant la revendication 1, caractérisé en ce qu'on utilise comme réactifs de racémisation dans la conduite de la deuxième étape, des hydroxydes de métaux alcalins ou de métaux alcalino-terreux ayant une teneur en eau comprise entre 0,1 et 30 % en poids.

7. Procédé suivant la revendication 1, caractérisé en ce qu'on utilise comme acides dans la conduite de la troisième étape des acides inorganiques ou organiques ou des polymères acides, dans chaque cas en présence d'eau.

8. Procédé suivant la revendication 1, caractérisé en ce qu'on opère à des températures comprises entre 20°C et 200°C dans la conduite de la première étape, à des températures comprises entre 50°C et 250°C dans la conduite de la deuxième étape et à des températures comprises entre 20°C et 150°C dans la conduite de la troisième étape.
